# EUROPEAN PATENT APPLICATION

(11) **EP 1 763 189 A1**
(43) Date of publication of application: **14.03.2007**
(21) Application number: 05255478.9
(22) Date of filing: 07.09.2005
(51) Int. Cl.: H04L 25/49, H03K 7/04, A61B 5/00, G08C 19/16

(54) **Data communication method**

(71) Applicant: Jetfly Technology Limited, Kowloon, Hong Kong (CN)
(72) Inventor: Chan, Shu Nam, Kowloon Hong Kong (CN); Yeung, Man Fat, New Territories Hong Kong (CN); Chan, Kwong Fu, Kowloon Hong Kong (CN); Fung, Man Chun Hubert, Sheung Wan Hong Kong (CN)
(74) Representative: Howe, Steven

(57) **Abstract**

A data transmission method is disclosed as including the steps of (a) measuring signals of an event; (b) forming a first data packet with a number of data pulses wherein the time interval between at least two of the data pulses represents a first measurement of the event; (c) forming a second data packet with a number of data pulses wherein the time interval between at least two of the data pulses represents a second measurement of the event; (d) sending the first data packet; and (e) subsequently sending the second data packet; in which the time interval between a rising edge of a first data pulse of the first data packet and rising edge of a first data pulse of the second data packet is selected for representation of a datum to be transmitted, e.g. for identification of a transmitter-receiver pair of a transmitter and a receiver.

## Description

This invention relates to a data transmission method and, in particular, such a method suitable for telemetric interference-tolerant transmission of data pulses. This invention also relates to a telecommunication method adopting such a data transmission method.

Such devices as heart rate monitors, speedometers for bicycles, and cadences usually include a transmitter and a receiver. The transmitter detects and measures signals of an event, such as a heart beat, revolution of a wheel of a bicycle, or revolution of a pedal of a bicycle. The measured signals are then formed, possibly with other information, into data packets, i.e. groups of data pulses, for telemetric transmission to the receiver. Upon reception of such data packets, the receiver deciphers the data pulses, e.g. for output of the relevant data and/or information on a visual display unit.

Where a number of such transmitter-receiver pairs operate in close proximity with one another, data transmitted by a transmitter of a first transmitter-receiver pair may be received by a receiver of a second transmitter-receiver pair, thus adversely affecting the performance of such devices, and the reliability and accuracy of the data and/or information displayed by the receiver.

As the transmission of data pulses consumes electric power, various ways have also been proposed for reducing the number of pulses to be transmitted. However, this will have an adverse effect on the amount of information capable of being transmitted.

It is thus an objective of the present invention to provide a data transmission method and a telecommunication method in which the above shortcomings are mitigated, or at least to provide a useful alternative to the trade and public.

It is a further objective of the present invention to provide a data transmission method and a telecommunication method to reduce the chance that data transmitted by a transmitter of a first transmitter-receiver pair are received by a receiver of a second transmitter-receiver pair.

It is a yet further objective of the present invention to provide a data transmission method and a telecommunication method the number of pulses to be transmitted may be reduced without reducing the amount of information transmitted.

According to a first aspect of the present invention, there is provided a data transmission method, including the steps of (a) measuring signals of an event; (b) forming a first data packet with at least two data pulses wherein the time interval between said at least two data pulses represents a first measurement of said event; (c) forming a second data packet with at least two data pulses wherein the time interval between said at least two data pulses represents a second measurement of said event; (d) transmitting said first data packet; and (e) subsequently transmitting said second data packet; characterized in that the time interval between a pre-determined pulse of said first data packet and a pre-determined pulse of said second data packet is selected for representation of at least a datum to be transmitted.

According to a second aspect of the present invention, there is provided a telecommunication method including the step of (a) measuring signals of an event; (b) forming a first data packet with at least two data pulses wherein the time interval between said at least two data pulses represents a first measurement of said event; (c) forming a second data packet with at least two data pulses wherein the time interval between said at least two data pulses represents a second measurement of said event; (d) transmitting said first data packet; (e) subsequently transmitting said second data packet; and (f) receiving said first and second data packets; characterized in that the time interval between a pre-determined pulse of said first data packet and a pre-determined pulse of said second data packet is selected for representation of a datum to be transmitted.

An embodiment of the present invention will now be described, by way of example only, with reference to the accompanying drawings in which:
Fig. 1 shows the scheme of an existing telecommunication protocol;
Fig. 2 shows schematically data packets arranged according to the existing telecommunication protocol in Fig. 1;
Fig. 3 shows an exemplary data packet arranged according to the existing telecommunication protocol in Fig. 1;
Fig. 4 shows schematically data packets arranged according to a method of the present invention; and
Fig. 5 shows exemplary data packets arranged according to a method of the present invention.

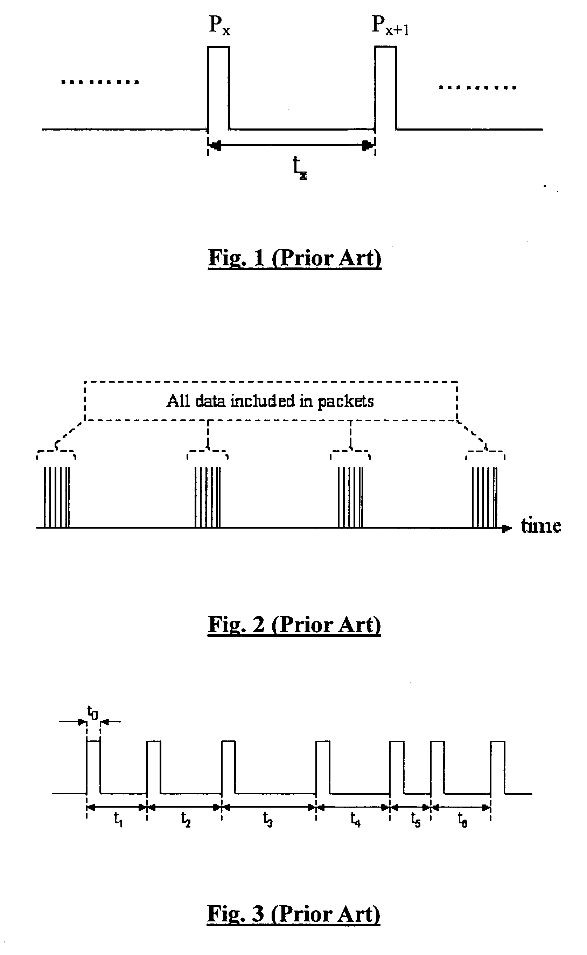

Some existing telecommunication protocols used for short-ranged communication systems make use of the time interval between successive data pulses to represent data. For example, as shown in Fig. 1, the time interval tₓ between the rising edge of a data pulse Pₓ and the rising edge of an immediately succeeding data pulse Pₓ₊₁ may be used for representing data to be transmitted by a transmitter for reception by a corresponding receiver.

As such transmitters usually only need to send data to the receiver occasionally, data are transmitted as packets (bursts of data), as shown in Fig. 2. In such a conventional protocol, all the data are contained within the data packets.

A sample packet for typical data transmission is defined in Table 1 below:

**Table 1**

| Module ID | Data 1 | Data 0 | User ID 1 | User ID 0 | Checksum |
|---|---|---|---|---|---|
| 4 bits | 4 bits | 4 bits | 4 bits | 4 bits | 4 bits |

Each packet of data contains six 4-bit information fields, further described as follows:
- Module ID: identification of transmitting devices with different functionality, such as heart rate monitors, speedometers, etc.;
- Data 1 and Data 0: provide an overall 8-bit data, such as numerical heart rate;
- User ID 1 and User ID 0: an overall 8-bit number for identifying transmitting devices of the same function;
- Checksum: error detection.

Turning now to Fig. 3, such shows an exemplary transmitted data packet in time domain. Each 4-bit information (binary '1' and '0') is encoded as the time interval (t₁, t₂, t₃, ... t₆) between the rising edges of successive data pulses, so that each packet includes seven pulses. While t₀ is fixed and is for identification of correct incoming pulse, whereas t₁ to t₆ may vary.

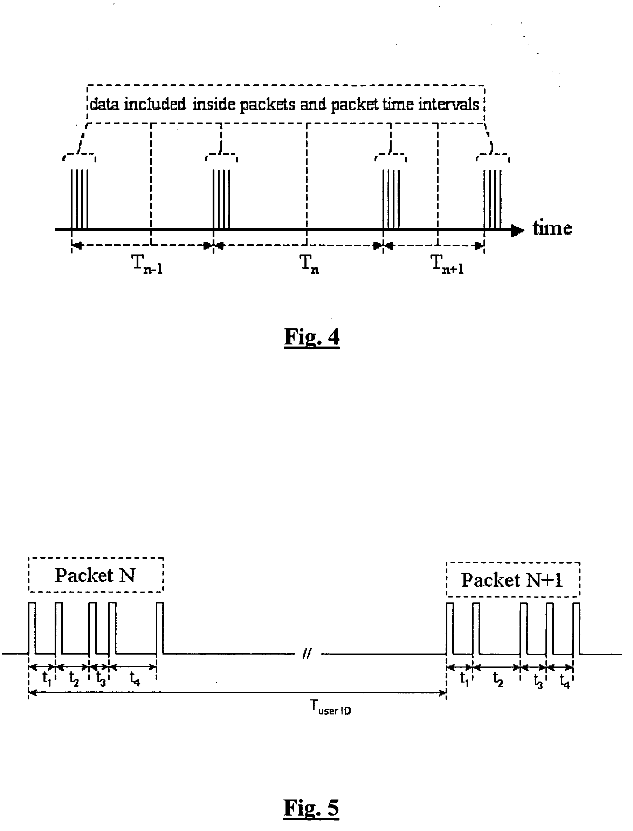

According to a method of the present invention, at least some of the data to be transmitted are encoded as the time interval between successive data packets (called "inter-packet time interval"), so that each data packet may contain less pulses. The inter-packet time interval may in general be defined as the time interval between the starting time (i.e. rising edge) of a pre-selected data pulse (e.g. the first data pulse, the second data pulse, or the last data pulse) of a data packet and the starting time of a pre-selected correspondingly positioned data pulse of the next succeeding data packet. For example, the inter-packet time interval may be defined as the starting time of the first data pulse of a data packet and the starting time of the first data pulse of the next succeeding data packet. Fig. 4 shows schematically data packets arranged according to a method of the present invention. In general, N different Tₙ can represent log ₂N bits of data.

As an example, the inter-packet time interval may be used for representing the 8-bit user ID. The resultant data packet will become shorter, as shown in Table 2 below:

**Table 2**

| Module ID | Data 1 | Data 0 | Checksum |
|---|---|---|---|
| 4 bits | 4 bits | 4 bits | 4 bits |

The corresponding transmitted packet in time domain is shown in Fig. 5. It can be seen that, for transmitting the same amount of data/information, the packet length is reduced from 24 bits to 16 bits, which is one-third shorter. As two data pulses can be eliminated from the original seven-pulse data packet (see Fig. 3), the power consumption is reduced by around 28.6%.

Although the method according to the present invention has so far been discussed in the context of modulating the user ID as the inter-packet time interval, it is of course readily understood by persons skilled in the art that the same method can be used for modulating other data or datum as the inter-packet time interval.

As a summary, such a protocol has at least the following two advantages:
1. The same amount of data/information can be transmitted by transmitting fewer data pulses. Thus, less power is consumed; and
2. The packet length is reduced, which in turn decreases turn-on time of the receiver and the chance of signal collision with other transmitter(s).

During a wireless data communication process, a transmitter of a transmitter-receiver pair will wirelessly transmit the data packets according to a method of the present invention. A receiver of a transmitter-receiver pair will sense and check the inter-packet time intervals of the received data packets to see if such correspond to a pre-selected time duration indicative of their originating from the transmitter of the same transmitter-receiver pair.

It should be understood that the above only illustrates an example whereby the present invention may be carried out, and that various modifications and/or alterations may be made thereto without departing from the spirit of the invention.

It should also be understood that certain features of the invention, which are, for clarity, described in the context of separate embodiments, may be provided in combination in a single embodiment. Conversely, various features of the invention which are, for brevity, described in the context of a single embodiment, may also be provided separately or in any appropriate sub-combinations.

## Claims

1. A data transmission method, including the steps of:
(a) measuring signals of an event;
(b) forming a first data packet with at least two data pulses wherein the time interval between said at least two data pulses represents a first measurement of said event;
(c) forming a second data packet with at least two data pulses wherein the time interval between said at least two data pulses represents a second measurement of said event;
(d) transmitting said first data packet; and
(e) subsequently transmitting said second data packet;
**characterized in that** the time interval between a pre-determined pulse of said first data packet and a pre-determined pulse of said second data packet is selected for representation of at least a datum to be transmitted.

2. A method according to Claim 1 further **characterized in that** the pre-determined pulse of said first data packet is the first data pulse in said data packet.

3. A method according to Claim 2 further **characterized in that** the pre-determined pulse of said second data packet is the first data pulse in said data packet.

4. A method according to Claim 1 further **characterized in that** the time interval between said pre-determined pulse of said first data packet and said pre-determined pulse of said second data packet is the time interval between the rising edge of said pre-determined pulse of said first data packet and the rising edge of said pre-determined pulse of said second data packet.

5. A method according to Claim 1 further **characterized in that** the datum represented by the time interval between said pre-determined pulse of said first data packet and said pre-determined pulse of said second data packet is for identification of a transmitter-receiver pair of a transmitter and a receiver.

6. A method according to Claim 1 wherein said steps (d) and (e) are carried out wirelessly.

7. A telecommunication method including the step of:
(a) measuring signals of an event;
(b) forming a first data packet with at least two data pulses wherein the time interval between said at least two data pulses represents a first measurement of said event;
(c) forming a second data packet with at least two data pulses wherein the time interval between said at least two data pulses represents a second measurement of said event;
(d) transmitting said first data packet;
(e) subsequently transmitting said second data packet; and
(f) receiving said first and second data packets;
**characterized in that** the time interval between a pre-determined pulse of said first data packet and a pre-determined pulse of said second data packet is selected for representation of a datum to be transmitted.

8. A method according to Claim 5 further **characterized in** including a step (g) of identifying said data packets as originating from said transmitter by sensing said time interval between said pre-determined pulse of said first data packet and said pre-determined pulse of said second data packet.
